# EUROPÄISCHE PATENTSCHRIFT

(11) **EP 0 487 985 B1**
(45) Veröffentlichungstag und Bekanntmachung des Hinweises auf die Patenterteilung: **17.04.1996**
(21) Anmeldenummer: 91119429.8
(22) Anmeldetag: 14.11.1991
(51) Int. Cl.: C12P 25/00, C12N 1/06

(54) **Verfahren zur Erhöhung des Riboflavin-Gehaltes in sprühgetrockneten Fermenterausträgen bei Riboflavin-Fermentationen**
Process for the enhancement of riboflavin levels in spray-dried riboflavinfermentation extracts
Procédé pour augmenter la teneur en riboflavine dans des extraits de fermentation de riboflavine

(30) Priorität: 24.11.1990 DE 4037441
(43) Veröffentlichungstag der Anmeldung: 03.06.1992
(73) Patentinhaber: BASF Aktiengesellschaft, D-67063 Ludwigshafen (DE)
(72) Erfinder: Kurth, Roland, Dr., W-6703 Limburgerhof (DE)

(56) Entgegenhaltungen:
- EP-A- 0 249 435
- WO-A-85/05126
- DE-A- 1 931 348
- FR-A- 2 250 823
- FR-A- 2 481 079
- CHEMICAL ABSTRACTS, vol. 93, no. 21, 24. November 1980, Columbus, OH (US); J. WILSKA-JESZKA et al., Seite 525, Nr. 202681d/

## Beschreibung

Die Erfindung betrifft die Erhöhung des Riboflavin-Gehaltes in sprühgetrockneten Fermenterausträgen bei Riboflavin-Fermentationen durch gezielte Autolyse der in der Fermentationslösung enthaltenen Zellen.

Die Herstellung von Riboflavin (Vitamin B2) durch mikrobielle Fermentationsprozesse ist bekannt. So kann Riboflavin z. B. gemäß DE 29 20 592 oder FR 2 250 823 durch Fermentation von Ashbya gossypii oder Eremothecium ashbyii, gemäß EP 211 289 durch Fermentation von zu Saccharomyces oder einer Variante davon gehörender Hefe, gemäß EP 231 605 mittels Candida flareri und gemäß DE 34 20 310 mittels Bacillus subtilis gewonnen werden.

Die Löslichkeit von Riboflavin in wäßrigen Lösungen liegt bei ca. 70 bis 200 mg Riboflavin pro Liter. In der Literatur sind Mikroorganismen beschrieben, die unter geeigneten Bedingungen über 5 g Riboflavin pro Liter Fermentationslösung in 5 Tagen produzieren. Der größte Teil des derart fermentativ hergestellten Riboflavins liegt somit in kristalliner Form in der Fermentationslösung vor. Da so hergestelltes Riboflavin hauptsächlich in der Tierernährung eingesetzt wird, ist es in vielen Fällen ausreichend, das kristalline Riboflavin samt der Biomasse durch Dekantieren oder Zentrifugieren aus den Fermenterausträgen abzutrennen und das erhaltene Sediment sprühzutrocknen. Auf diese weise kommt man zu Produkten mit Riboflavin-Gehalten von etwa 20 bis 60 Gew.-%. Für andere Verwendungszwecke, insbesondere für pharmazeutische Zwecke, muß Riboflavin weiter gereinigt werden.

So ist aus der DE-C 29 20 592 ein Verfahren zur Abtrennung von Riboflavin aus Fermentersuspensionen bekannt, bei welchem die Fermentationssuspensionen mit 25 bis 100 Vol-% Wasser verdünnt und anschließend für 15 bis 45 Minuten zwecks Öffnung der Riboflavin enthaltenden Zellen auf 50 bis 65°C erwärmt werden. Nach dem Abkühlen der Suspension werden diese zur Anreicherung von Riboflavin zweimal zentrifugiert. Zur Erhöhung des Riboflavin-Gehaltes wird empfohlen, der verdünnten Brühe während des Erwärmens oder vorzugsweise nach dem Erwärmen ein proteolytisches Enzym zuzusetzen und dieses 3 bis 4 Stunden einwirken zu lassen. Als Beispiele für geeignete Enzyme werden alkalische Proteasen, wie B. subtilis-Protease und B. ligninoformis-Protease oder neutrale Proteasen, wie B. subtilis-Proteasen genannt. Solche Enzyme sind im Handel erhältlich. Nachteilig an dem Verfahren gemäß DE 29 20 592 ist, daß man infolge der Verdünnung ein beträchtlich größeres aufzuarbeitendes Volumen an Fermentersuspensionen erhält, wodurch die Kosten der Aufarbeitung und die Riboflavinverluste durch das im zugesetzten Wasser gelöste Riboflavin erhöht werden muß. Außerdem sind die mitverwendeten proteolytischen Enzyme relativ teuer.

Es war daher die Aufgabe der Erfindung ein Verfahren zu entwickeln, mit dessen Hilfe der Riboflavin-Gehalt in sprühgetrockneten Fermenterausträgen bei Riboflavin-Fermentationen auf möglichst einfache und wirtschaftliche Weise erhöht wird und der Verlust an Riboflavin während der Aufarbeitung minimiert wird.

Es wurde nun gefunden, daß sich die gezielte Autolyse von Riboflavin produzierenden Mikroorganismen zur Herstellung eines sprühgetrockneten Produktes mit einem höheren Riboflavin-Gehalt nutzen läßt.

Gegenstand der Erfindung ist dementsprechend ein Verfahren zur Erhöhung des Riboflavin-Gehaltes in sprühgetrockneten Fermenterausträgen bei Riboflavin-Fermentationen, das dadurch gekennzeichnet ist, daß man in der maximale Ausbeuten an Riboflavin enthaltenden Fermentationslösung die Auflösung der enthaltenen Zellen durch gezielte Bildung oder Aktivierung der intrazellularen Zellwand-abbauenden- und Protein-abbauenden Enzyme induziert, d.h. eine Autolyse induziert.

Unter induzierter Autolyse versteht man die Bildung und Aktivierung der eigenen intrazellularen Zellwand-abbauenden und Protein-abbauenden Enzyme. Die praktische Anwendung dieser Methode wurde bisher nur zur Vereinfachung der Herstellung von Proteinextrakten aus Hefefermentationen beschrieben. Bei dem bekannten Prozeß wird erst durch induzierte Autolyse das gewünschte Produkt gebildet.

Eine solche Gewinnung von Bierhefeextrakten oder Weinhefeextrakten wird beispielsweise in Chem. Abstr. 93 (1980) Abstr. No. 202681d bzw. in FR-A-2 481 079 beschrieben. Die hier beschriebenen Extrakte enthalten auch geringe Mengen an Vitaminen der B-Gruppe.

Weiterhin ist aus DE-A-19 31 348 bekannt, daß man durch ein Autolyseverfahren die bei der Bierherstellung als Nebenprodukt anfallende Brauereihefe in eine Bouillon aus abgetöteten Hefezellen überführen kann. Diese kann dann als leicht verdauliches Viehfutter verwendet werden, das auch geringe Mengen an Riboflavin enthält.

Die Autolyse von Mikroorganismen kann durch sogenannte Streßfaktoren in den Mikroorganismen induziert werden.

Solche Streßfaktoren für Mikroorganismen können beispielsweise sein: Nährstoffmangel, Temperaturerhöhungen, Sauerstoffmangel, pH-Wert-Veränderungen sowie Änderungen des Druckes oder der Osmolarität im Medium, aber auch die Zugabe von Salzen, Tensiden oder Fettsäuren zum Medium oder aber auch Bestrahlung mit UV-Licht oder Röntgenstrahlen (vgl. Acta Biotechnol. 5 (1985) 2, 129-136).

Als Riboflavin-bildende Mikroorganismen können beispielsweise der Produktionsstamm Ashbya gossypii und Mutanten hiervon, der Produktionsstamm Eremothecium ashbyii und Mutanten hiervon, Mikroorganismen der Gattungen Candida, Torulopsis, Bacillus oder Saccharomyces verwendet werden.

Als besonders geeignete Streßfaktoren seien die Temperaturerhöhung unter Sauerstoffmangel genannt.

So lassen sich beispielsweise die Riboflavin-bildenden Stämme Ashbya gossypii und Mutanten hiervon bei Temperaturen von 35 bis 55°C innerhalb von 6 bis 28 Stunden unter fast vollständigem Ausschluß von Sauerstoff, d.h. beim Erwärmen ohne Belüften (Durchleiten von Luft) lysieren. Der pH-Wert der Lösung sollte hierbei zwischen 4 und 9 liegen.

Gegenstand der Erfindung ist daher auch ein Verfahren zur Erhöhung des Riboflavin-Gehaltes in sprühgetrockneten Fermenterausträgen bei der fermentativen Herstellung von Riboflavin mittels Riboflavin produzierender Microorganismen, dadurch gekennzeichnet, daß man die maximale Ausbeuten an Riboflavin enthaltende Fermentationslösung bei einem pH-Wert zwischen 4 und 9, vorzugsweise zwischen 5 und 8, insbesondere zwischen 6 und 7 und unter weitgehendem Ausschluß von Sauerstoff, d.h. ohne Belüftung, für 6 bis 28 Stunden, vorzugsweise 14 bis 24 Stunden auf Temperaturen von 35 bis 55°C, vorzugsweise 40 bis 49°C, insbesondere 40 bis 48°C erwärmt, anschließend das in kristalliner Form vorliegende Riboflavin durch Dekantieren oder Zentrifugieren abtrennt und das erhaltene Sediment sprühtrocknet.

Es wird zwar in der oben ausführlich gewürdigten DE 29 20 592 schon angegeben, daß man die Riboflavin enthaltende Fermentationsbrühe für 15 bis 45 Minuten auf Temperaturen von 50 bis 65°C erwärmen soll und daß dies Erwärmen zur Lyse der Zellen und zur Verminderung der Viskosität der Brühe dient, jedoch handelt es sich hierbei um eine ganz andere Wirkung. Durch so kurzzeitige Wärmebehandlung werden lediglich die das Riboflavin enthaltenden Zellen aufgebrochen, wobei der Zellinhalt ausfließt. Demgegenüber erfolgt durch die erfindungsgemäße mehrstündige Inkubation der Fermentationslösung bei tieferen Temperaturen ein vollständiger enzymatischer Abbau der festen Zellbestandteile, d.h. Zellen und Zellwände sind im mikroskopischen Bild der Lösung nach der Autolyse nicht mehr zu erkennen (vgl. Beispiel 5a-d). Oberhalb von etwa 55°C, teilweise auch schon ab 50°C, werden die zur erfindungsgemäßen Autolyse benötigten intrazellularen

Zellwand-abbauenden und Protein-abbauenden Enzyme irreversibel zerstört.

Das erfindungsgemäß verwendete Ausgangsmaterial ist eine Riboflavin enthaltende Fermentationslösung. Die Herstellung von Riboflavin durch Fermentation bzw. Gärung ist bekannt. Kurz gesagt wird bei dieser Methode ein Nährmedium sterilisiert und mit einem zur Bildung von Riboflavin befähigten Mikroorganismus, wie Ashbya gossypii oder Eremothecium ashbyii beimpft und inkubiert. Wenn die Fermentationsausbeute an Riboflavin an den Maximalwert herankommt oder diesen Wert etwa erreicht, wird die Fermentation beendet. Nach der erfindungsgemäßen induzierten Autolyse der Fermentationslösung kann das Riboflavin in üblicher Weise durch Zentrifugieren oder Dekantieren und anschließendes Sprühtrocknen des Sediments erhalten werden.

Das Sprühtrocknen des Sediments kann durch einfaches Einsprühen mittels einer Zweistoffdüse in einem Trockenturm erfolgen. Ein besser handhabbares Produkt erhält man jedoch, wenn man das Sediment einer Sprühwirbelschichttrocknung oder aber einer Einstoffdüsenzerstäubungstrocknung unterwirft, wie sie in DE-A 38 19 745 beschrieben sind. Mit besonderem Vorteil arbeitet man in einem Sprühtrockner mit integriertem Fließbett (Abkürzung: FSD = Fluidized Spray Dryer), wie er in Chem.-Ing.-Technik 59 (1987) Nr. 2, Seiten 112-117, insbesodnere Seite 115, beschrieben ist.

Durch das erfindungsgemäße Verfahren kann der Riboflavin-Gehalt in den sprühgetrockneten Fermenterausträgen im allgemeinen um etwa 20 %, in besonderen Fällen sogar um bis zu 45 % erhöht werden.

Die folgenden Beispiele sollen das erfindungsgemäße Verfahren verdeutlichen.

### Beispiel 1

Nach Beendigung einer Riboflavin-Fermentation unter Verwendung eines Stammes von Ashbya gossypii wurde die erhaltene Fermentationslösung mit einem Riboflavin-Gehalt von > 5 g/l unter leichtem Rühren und ohne Einleiten von Luft bei einem pH-Wert von etwa 6,5 für 17 Stunden (h) auf die aus der folgenden Tabelle 1 angegebenen Temperaturen erwärmt. Anschließend wurde die erhaltene Suspension mikroskopisch untersucht. Die erhaltenen Ergebnisse sind in der folgenden Tabelle 1 zusammengestellt.

**Tabelle 1**

| Beispiel | Temperatur | im mikroskopischen Bild neben Riboflavin Sichtbares |
|---|---|---|
| 1a | 30°C | viele Zellen |
| 1b | 35°C | viele Zellen |
| 1c | 40°C | wenige Zellen |
| 1d | 45°C | keine Zellen |
| 1e | 50°C | keine Zellen |
| 1f | 60°C | viele Zellen und Zellhülsen |
| 1e | 70°C | viele Zellen und Zellhülsen |

Das Versuchsergebnis zeigt, daß durch 17-stündiges Erwärmen des Fermenteraustrages auf 45°C oder 50°C bei einem pH-Wert von 6,5, leichtem Rühren und quasi anaeroben Bedingungen eine vollständige Autolyse der in der Fermentationslösung enthaltenen Zellen und Zellwände hervorgerufen wird.

### Beispiel 2

Nach Beendigung einer Riboflavin-Fermentation, bei der in der Fermentationslösung ein Riboflavin-Gehalt von > 5 g/l erzielt worden war, wurde der Fermenteraustrag bei den aus der folgenden Tabelle 2 angegebenen pH-Werten unter leichtem Rühren und ohne Einleiten von Luft für 17 h auf 45°C erwärmt. Anschließend wurden die Feststoffbestandteile der so erwärmten Fermentationslösung in einer Laborzentrifuge quantitativ sedimentiert. Das Sediment wurde anschließend bis zur Gewichtskonstanz getrocknet und der Riboflavin-Gehalt des getrockneten Sediments bestimmt. Zum Vergleich (Beispiel 2i) wurde der unbehandelte Fermentationsaustrag quantitativ sedimentiert, das Sediment getrocknet und darin der Riboflavin-Gehalt bestimmt.

**Tabelle 2**

| Beispiel | pH-Wert | Riboflavin-Gehalt im getrockneten Sediment [Gew.-%] |
|---|---|---|
| 2a | 4,0 | 20 |
| 2b | 5,0 | 25 |
| 2c | 6,0 | 32 |
| 2d | 7,0 | 42 |
| 2e | 8,0 | 34 |
| 2f | 9,0 | 28 |
| 2g | 10,0 | 20 |
| 2i (zum Vergleich) | unbehandelte Fermentationslösung | 20 |

### Beispiel 3

Nach Beendigung einer Riboflavin-Fermentation in einem 2000 l-Fermenter, bei der ein Riboflavin-Gehalt von > 5 g/l erhalten worden war, wurde der Fermentationsaustrag halbiert. Die eine Hälfte wurde direkt, d.h. ohne Autolyse dekantiert und sprühgetrocknet und die andere Hälfte wurde unter quasi anaeroben Bedingungen (leichtes Rühren ohne Einleiten von Luft) bei einem pH-Wert von 6,8 für 24 h bei 45°C inkubiert. Anschließend wurde die autolysierte Fermentationslösung dekantiert und sprühgetrocknet. Die erzielten Ergebnisse sind in der folgenden Tabelle 3 angegeben.

**Tabelle 3**

| Beispiel | Fermentationsaustrag | Riboflavin-Gehalt im sprühgetrockneten Sediment [Gew.-%] |
|---|---|---|
| 3a | ohne anschließende Autolyse sprühgetrocknet | 60 |
| 3b | mit anschließender Autolyse sprühgetrocknet | 90 |

### Beispiel 4

Nach Beendigung einer Riboflavin-Fermentation unter Verwendung eines Stammes von Ashbya gossypii wurde die erhaltene Fermentationslösung mit einem Riboflavingehalt von > 5 g/l unter leichtem Rühren und ohne Einleiten von Luft bei einem pH-Wert von 6,5 bei 45°C inkubiert. Im Abstand von 2 bis 4 h wurden Proben entnommen und untersucht. Hierzu wurden jeweils 45 ml bei 2200 Umdrehungen pro Minute 5 Minuten lang in einer Laborzentrifuge zentrifugiert. Das Sediment wurde in Saline (0,9 %ige wäßrige NaCl-Lösung) im gleichen Volumen resuspendiert und erneut wie oben angegeben zentrifugiert. Anschließend wurde das Sediment lyophilisiert. Die erzielten Ergebnisse sind in Tabelle 4 angegeben.

**Tabelle 4**

| Beispiel | Dauer der Autolyse in Stunden [h] | Riboflavin-Gehalt im lyophilisierten Sediment [Gew.-%] |
|---|---|---|
| 4a | 0 | 45 |
| 4b | 4 | 50 |
| 4c | 6 | 56 |
| 4d | 8 | 64 |
| 4e | 10 | 71 |
| 4f | 12 | 75 |
| 4g | 14 | 78 |
| 4h | 16 | 81 |
| 4i | 20 | 85 |
| 4j | 24 | 88 |
| 4k | 28 | 91 |

### Beispiel 5

Gegenüberstellung der Wirkung des Erwärmens der Fermentationslösung gemäß DE 29 20 592 (Beispiele 5a-5c) und der Wirkung der erfindungsgemäßen Autolyse.

Nach Beendigung einer Riboflavin-Fermentation mit Ashbya gossypii wurde die erhaltene Fermentationslösung mit einem Riboflavin-Gehalt von mehr als 5 g/l aufgeteilt und unter den aus Tabelle 5 ersichtlichen Bedingungen inkubiert.

Die Proben wurden anschließend in einer Laborzentrifuge zentrifugiert, das Sediment 2x mit Saline gewaschen, resuspendiert und erneut zentrifugiert. Anschließend wurde das Sediment lyophilisiert. Die erhaltenen Ergebnisse sind in Tabelle 5 angegeben.

**Tabelle 5**

| Beispiel | Inkubationsbedingungen | | mikroskopisches Bild | Riboflavin-Gehalt im lyophilisierten Sediment [Gew.-%] |
|---|---|---|---|---|
| | Dauer | Temperatur | | |
| 5a (z.Vgl.) | 45 min | 20°C | viele Zellen sichtbar | 27 |
| 5b (z.Vgl.) | 45 min | 50°C | viele Zellen und Zellwände sichtbar | 29 |
| 5c (z.Vgl.) | 45 min | 65°C | viele Zellen und Zellwände sichtbar | 32 |
| 5d | 20 h | 45°C | keine Zellen und Zellwände | 81 |

Durch das Erwärmen für 45 Minuten auf 50 bis 65°C wird eine Lyse der Zellen von Ashbya gossypii - wie in DE 29 20 592 beschrieben - erreicht. Dabei wird unter Lyse das Aufbrechen der Riboflavin enthaltenden Zellen verstanden, wobei der Zellinhalt ausfließt. Im mikroskopischen Bild sind daher die Zellwände (vgl. Beispiel 5b und 5c) noch vollständig erhalten und die Biomasse im lyophilisierten Sediment enthalten. Demgegenüber erfolgt unter den erfindungsgemäßen Bedingungen der Autolyse (vgl. Beispiel 5d) der vollständige enzymatische Abbau der Zellen, d.h. im mikroskopischen Bild der Fermentationslösung sind keine Zellen oder Zellwände mehr sichtbar.

## Patentansprüche

1. Verfahren zur Erhöhung des Riboflavin-Gehaltes in sprühgetrockneten Fermenterausträgen bei der fermentativen Herstellung von Riboflavin mittels Riboflavin produzierender Mikroorganismen, dadurch gekennzeichnet, daß man die maximale Ausbeuten an Riboflavin enthaltende Fermentationslösung bei einem pH-Wert zwischen 4 und 9 unter weitgehendem Ausschluß von Sauerstoff, d.h. ohne Belüftung, für 6 bis 28 Stunden auf Temperaturen von 35 bis 55°C erwärmt, anschließend das in kristalliner Form vorliegende Riboflavin durch Dekantieren oder Zentrifugieren abtrennt und das erhaltene Sediment sprühtrocknet.

2. Verfahren gemäß Anspruch 1, dadurch gekennzeichnet, daß man die Riboflavin enthaltende Fermentationslösung bei einem pH-Wert zwischen 6 und 7 unter weitgehendem Ausschluß von Sauerstoff auf Temperaturen von 40 bis 49°C erwärmt.

3. Verfahren gemäß Anspruch 1, dadurch gekennzeichnet, daß man die Riboflavin enthaltende Fermentationslösung auf Temperaturen von 40 bis 48°C erwärmt.

4. Verfahren gemäß Anspruch 1, dadurch gekennzeichnet, daß man die Riboflavin enthaltende Fermentationslösung für 14 bis 24 Stunden auf Temperaturen von 35 bis 55°C erwärmt.

## Claims

1. A process for increasing the riboflavin content in spray-dried discharges from fermenters in the preparation of riboflavin by fermentation using riboflavin-producing microorganisms, which comprises heating the fermentation solution, which contains maximal yields of riboflavin, at a pH of from 4 to 9 with substantial exclusion of oxygen, ie. without aeration, at from 35 to 55°C for from 6 to 28 hours, then removing the riboflavin present in crystalline form by decantation or centrifugation, and spray-drying the resulting sediment.

2. A process as claimed in claim 1, wherein the riboflavin-containing fermentation solution is heated at a pH of from 6 to 7 with substantial exclusion of oxygen at from 40 to 49°C.

3. A process as claimed in claim 1, wherein the riboflavin-containing fermentation solution is heated at from 40 to 48°C.

4. A process as claimed in claim 1, wherein the riboflavin-containing fermentation solution is heated at from 35 to 55°C for from 14 to 24 hours.

## Revendications

1. Procédé d'augmentation de la teneur en riboflavine dans des produits de fermenteur séchés par pulvérisation, lors de la préparation par fermentation de la riboflavine, au moyen de micro-organismes produisant de la riboflavine, caractérisé en ce que l'on chauffe la solution de fermentation, contenant la quantité produite maximale de riboflavine, à un pH compris entre 4 et 9, avec un absence poussée d'oxygène, c'est-à-dire sans aération, pendant une durée de 6 à 28 heures, à des températures allant de 35 à 55°C, puis on effectue la séparation de la riboflavine, présente sous forme cristalline, par décantation ou centrifugation et l'on sèche par pulvérisation le sédiment obtenu.

2. Procédé selon la revendication 1, caractérisé en ce qu'on chauffe la solution de fermentation contenant la riboflavine à un pH compris entre 6 et 7 avec une absence poussée d'oxygène à des températures allant de 40 à 49°C.

3. Procédé selon la revendication 1, caractérisé en ce qu'on chauffe la solution de fermentation contenant la riboflavine à des températures allant de 40 à 48°C.

4. Procédé selon la revendication 1, caractérisé en ce qu'on chauffe la solution de fermentation contenant la riboflavine pendant 14 à 24 heures à des températures allant de 35 à 55°C.
